# EUROPÄISCHE PATENTSCHRIFT

(11) **EP 0 645 376 B1**
(45) Veröffentlichungstag und Bekanntmachung des Hinweises auf die Patenterteilung: **07.04.1999**
(21) Anmeldenummer: 94114519.5
(22) Anmeldetag: 15.09.1994
(51) Int. Cl.: C07D 263/24, C07D 413/12, A61K 31/42

(54) **Substituierte 1-Phenyl-oxazolidin-2-on Derivate deren Herstellung und der Verwendung als Adhäsionsrezeptor-Antagonisten**
Substituted 1-phenyl-oxazolidin-2-one derivatives, their preparation and their use as adhesion-receptor antagonists
Dérivés de 1-phényl-oxazolidin-2-one substitués, leur préparation et leur utilisation comme antagonistes du récepteur d'adhésion

(30) Priorität: 23.09.1993 DE 4332384
(43) Veröffentlichungstag der Anmeldung: 29.03.1995
(73) Patentinhaber: MERCK PATENT GmbH, 64293 Darmstadt (DE)
(72) Erfinder: Gante, Joachim, Prof. Dr., D-64291 Darmstadt (DE); Juraszyk, Horst, Dr., D-64342 Seeheim (DE); Raddatz, Peter, Dr., D-64342 Seeheim (DE); Wurziger, Hanns, Dr., D-64291 Darmstadt (DE); Melzer, Guido, D-65719 Hofheim/Ts. (DE); Bernotat-Danielowski, Sabine, Dr., D-61231 Bad Nauheim (DE)

(56) Entgegenhaltungen:
- EP-A- 0 300 272
- EP-A- 0 381 033
- EP-A- 0 443 197
- EP-A- 0 605 729
- EP-A- 0 623 615
- EP-A- 0 635 505
- JOURNAL OF MEDICINAL CHEMISTRY, Bd.35, Nr.23, 13. November 1992 Seiten 4393 - 4407 ALIG L. ET AL. 'Low molecular weight, non-peptide fibrinogen receptor antagonists'
- JOURNAL OF MEDICINAL CHEMISTRY, Bd.36, Nr.13, 25. Juni 1993 Seiten 1811 - 1819 ZABLOCKI J.A. ET AL. 'Potent in vitro and in vivo inhibitors of platelet aggregation based upon the Arg-Gly-Asp-Phe sequence of fibrinogen. A proposal on the nature of the binding interaction between the Arg-guanidine of RGDX mimetics and the platelet GP IIb-IIIa receptor'

## Beschreibung

Die Erfindung betrifft Oxazolidinonderivate der Formel I 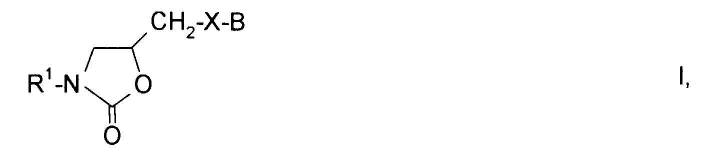 worin
- R¹: einen einfach durch H₂N-CH₂-, A₂N-CH₂-, H₂N-C(=NH)-, H₂N-C(=NH)-NH-, H₂N-C(=NH)-NH-CH₂-, HO-NH-C(=NH)- oder HO-NH-C(=NH)-NH- substituierten Phenylrest,
- X: O,
- B: 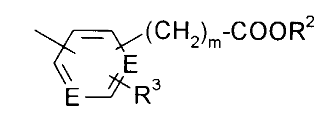
- A: Alkyl mit 1 bis 6 C-Atomen,
- R²: H, A, Li, Na, K, NH₄ oder Benzyl,
- R³: H oder (CH₂)ₙ-COOR²,
- E: CH
- m: 1, 2 oder 3 und
- n: 0, 1, 2 oder 3 bedeuten,
sowie deren physiologisch unbedenkliche Salze.

Ähnliche Verbindungen sind aus der EP-A1-0 381 033 bekannt.

Oxazolidinonderivate mit anderen Substituenten und anti-hyperlipidämischen Eigenschaften sind in der EP 0 605 729 beschrieben.

Andere Oxazolidinone, jedoch mit Wirkungen auf das Zentralnervensystem, sind z.B. aus der EP 0 300 272 und der EP 0 443 197 bekannt.

Peptide, die als Fibrinogenrezeptor-Antagonisten wirken, sind z.B. in J. Med. Chem. (1992) 35, 4393-4407 und in J. Med. Chem. (1993) 36, 1811-1819, beschrieben.

Oxazolidinonderivate, die die Bindung von Fibrinogen an den Fibrinogenrezeptor hemmen, jedoch andere Substituenten aufweisen, sind aus der EP 0 623 615 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Diese Aufgabe wurde durch die Erfindung gelöst. Es wurde gefunden, daß die Verbindungen der Formel I sowie ihre Solvate und Salze bei guter Verträglichkeit wertvolle pharmakologische Eigenschaften besitzen. Insbesondere hemmen sie die Bindung von Fibrinogen, Fibronectin und des von Willebrand-Faktors an den Fibrinogenrezeptor der Blutplättchen (Glykoprotein llb/llla) als auch die Bindung derselben und weiterer adhäsiver Proteine, wie Vitronectin, Kollagen und Laminin, an die entsprechenden Rezeptoren auf der Oberfläche verschiedener Zelltypen. Die Verbindungen beeinflussen somit Zell-Zell- und Zell-Matrix-Wechselwirkungen. Sie verhindern insbesondere die Entstehung von Blutplättchenthromben und können daher zur Behandlung von Thrombosen, Apoplexie, Herzinfarkt, Entzündungen, Arteriosklerose verwendet werden. Ferner haben die Verbindungen einen Effekt auf Tumorzellen, indem sie deren Metastasierung hemmen. Somit können sie auch als Anti-Tumor-Mittel eingesetzt werden.

Die Eigenschaften der Verbindungen können nach Methoden nachgewiesen werden, die in der EP-A1-0 462 960 beschrieben sind. Die Hemmung der Fibrinbindung an den Fibrinogenrezeptor kann nach der Methode nachgewiesen werden, die in der EP-A1-0 381 033 angegeben ist. Die thrombozytenaggregationshemmende Wirkung läßt sich in vitro nach der Methode von Born (Nature 4832, 927-929, 1962) nachweisen.

Gegenstand der Erfindung ist ferner ein Verfahren zur Herstellung einer Verbindung der angegebenen Formel I sowie von deren Salzen, dadurch gekennzeichnet, daß man eine Verbindung der Formel II 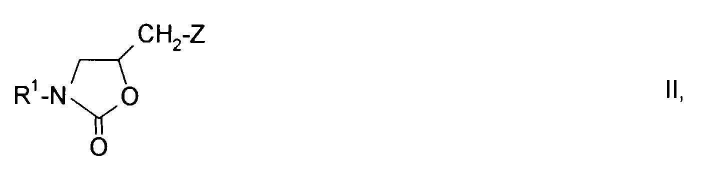 worin
- R¹: die oben angegebene Bedeutung hat
und
- Z: Cl, Br, I, OH oder eine reaktionsfähig veresterte OH-Gruppe bedeutet,
mit einer Verbindung der Formel III

Y-B III,

worin
- B: die oben angegebene Bedeutung hat
und
- Y: OH, SH, NH₂, NAH oder einen aus OH oder SH ableitbaren salzartigen Rest bedeuten,
umsetzt, oder daß man
eine Verbindung der Formel IV

R¹-NH-CH₂-CH(OH)-CH₂-X-B IV,

worin
R¹, B und X die oben angegebenen Bedeutungen haben, oder eines ihrer reaktionsfähigen Derivate mit einem reaktiven Derivat der Kohlensäure umsetzt,
oder daß man zur Herstellung einer Guanidinoverbindung der Formel I (R¹ = ein einfach durch H₂N-C(=NH)-NH- substituierter Phenylrest) eine Aminoverbindung entsprechend der Formel I, die jedoch anstelle des Restes R¹ eine Aminophenylgruppe enthält, mit einem amidinierenden Mittel behandelt,
oder daß man eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder beide Reste R¹ und/oder B in (einen) andere(n) Rest(e) R¹ und/oder B umwandelt und/oder eine Verbindung der Formel I durch Behandeln mit einer Säure oder einer Base in eines ihrer Salze überführt.

Die Verbindungen der Formel I besitzen mindestens ein chirales Zentrum und können daher in mehreren enantiomeren Formen auftreten. Alle diese Formen (z.B. D- und L-Formen) und deren Gemische (z.B. die DL-Formen) sind in der Formel 1 eingeschlossen.

Vor- und nachstehend haben die Reste bzw. Parameter B, X, R¹ bis R³, A, E, Y, Z, m und n die bei den Formeln I, II oder III angegebenen Bedeutungen haben, falls nicht ausdrücklich etwas anderes angegeben ist.

In den vorstehenden Formeln hat die Gruppe A 1-6, vorzugsweise 1,2, 3 oder 4 C-Atome. Im einzelnen bedeutet A vorzugsweise Methyl, Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl.

X ist O.

R¹ ist vorzugsweise ein in 4-Stellung, aber auch in 2- oder 3-Stellung wie oben angegeben substituierter Phenylrest, im einzelnen bevorzugt 2-, 3- oder insbesondere 4-Amidinophenyl; 2-, 3- oder 4-Aminomethylphenyl; 2-, 3- oder 4-Guanidinomethylphenyl; oder aber 2-, 3- oder 4-N-Alkylaminomethylphenyl, wobei in diesen Fällen Alkyl vorzugsweise für Methyl oder Ethyl steht.

B ist vorzugsweise ein- oder zweifach substituiertes Phenyl, wobei die genannten Substituenten möglich sind. Im einzelnen steht B bevorzugt für 2-, 3- oder 4-Carboxymethyl-, 2-, 3- oder 4-Methoxycarbonyl- oder -Ethoxycarbonyl-phenyl, ferner auch vorzugsweise für 2,3-, 2,4-, 2,5-, 2,6-, 3,4-, 3,5-Dicarboxymethylphenyl sowie vorzugsweise auch für die Methyl- oder Ethylester der zuvor genannten bevorzugten Reste und auch für die daraus ableitbaren Li-, Na-, K- oder Ammoniumsalzreste.

R² bedeutet vorzugsweise Wasserstoff, A oder Na, während R³ besonders bevorzugt für H oder Carboxymethyl steht.

Die Parameter m und n sind bevorzugt 1, ferner aber auch 2 oder 3. Die Variable n kann darüber hinaus auch 0 sein.

Unter den Verbindungen der Formel I sind diejenigen bevorzugt, in denen mindestes einer der angegebenen Reste, Gruppen und/oder Parameter eine der angegebenen bevorzugten Bedeutungen hat. Einige Gruppen von bevorzugten Verbindungen sind diejenigen der Formeln la bis Ih, die der Formel I entsprechen, worin jedoch

| | | |
|---|---|---|
| in Ia | X | O bedeutet, |
| | | |
| in Ib | X | O und |
| | B | 2-, 3- oder 4-Carboxymethylphenyl bedeutet; |
| | | |
| in Ic | X | O und |
| | R¹ | 2-, 3- oder 4-Amidinophenyl bedeutet; |
| | | |
| in Id | X | O und |
| | B | 2,3-, 2,4-, 2,5-, 2,6-, 3,4- oder 3,5-Dicarboxymethylphenyl bedeutet; |
| | | |
| in Ie | X | O und |
| | B | 2-Carboxymethyl- oder 3-Carboxymethyl-thien-4-yl oder -pyrrol-4-yl bedeutet; |
| | | |
| in If | X | O und |
| | B | 2,3- oder 2,5-Dicarboxymethyl- oder 2-Carboxymethyl-3-carboxy- bzw. 2-Carboxymethyl-5-carboxy-pyrrol-4-yl bedeutet; |
| | | |
| in Ig | X | O, |
| | B | 2-, 3- oder 4-Carboxyphenyl und |
| | R¹ | 2, 3- oder 4-Amidinophenyl bedeutet; |
| | | |
| in Ih | X | O, |
| | B | 2,3-, 2,4-, 2,5-, 2,6-, 3,4- oder 3,5-Dicarboxymethylphenyl und |
| | R¹ | 2-, 3- oder 4-Amidinophenyl bedeutet. |

Ferner sind bevorzugt Verbindungen, die an sich den Formeln la bis Ih entsprechen, worin aber die Carboxygruppe des Restes B durch eine Methoxycarbonyl- oder Ethoxycarbonylgruppe ersetzt ist.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner EP-A1-0381033, EP-A1-0462960) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die an Stelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl (DNP)), Aralkoxymethyl- (z.B. Benzyloxymethyl (BOM)) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl, tert.-Butoxycarbonyl (BOC), 2-Jodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden hergestellt werden, wie sie z.B. in den genannten Standardwerken und Patentanmeldungen beschrieben sind, z.B. durch Umsetzung von Verbindungen, die den Formeln II und III entsprechen, wobei jedoch mindestens eine dieser Verbindungen eine Schutzgruppe anstelle eines H-Atoms enthält.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Dioxan bei 15-60° abgespalten weden, die FMOC-Gruppe mit einer etwa 5-20%igen Lösung von Dimethylamin, Diethylamin oder Piperidin in DMF bei 15-50°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Verbindungen der Formel I können bevorzugt auch durch Reaktion eines Oxazolidinons der Formel II mit einer Verbindung der Formel III erhalten werden. Dabei bedient man sich zweckmäßig der an sich bekannten Methoden der Veretherung oder der N-Alkylierung von Aminen.

Die Abgangsgruppe Z der Formel II bedeutet vorzugsweise Cl, Br, I, C₁- bis C₆-Alkylsulfonyloxy wie Methan- oder Ethansulfonyloxy oder C₆-C₁₀-Arylsulfonyloxy wie Benzol-, p-Toluol- oder 1- oder 2-Naphthalinsulfonyloxy.

Die Reaktion gelingt vorzugsweise in Gegenwart einer zusätzlichen Base, z.B. eines Alkali- oder Erdalkalimetall-hydroxids oder carbonats wie Natrium-, Kalium- oder Calciumhydroxid, Natrium-, Kalium- oder Calciumcarbonat, in einem inerten Lösungsmittel z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 200, vorzugsweise zwischen 0 und 120°. Falls die Fluchtgruppe Z von I verschieden ist, empfiehlt sich ein Zusatz eines Iodids wie Kaliumiodid.

Die Ausgangsstoffe der Formel II sind in der Regel neu. Sie können z.B. hergestellt werden durch Reaktion eines substituierten Anilins der Formel R¹-NH₂, worin R¹ die angegebene Bedeutung hat, mit einer Verbindung der Formel R⁵CH₂-CHR⁶-CH₂OH (worin R⁵ Z, R⁶ OR⁷, R⁷ eine Schutzgruppe, R⁵ und R⁶ zusammen auch O bedeuten) zu einer Verbindung der Formel R¹-NH-CH₂-CHR⁸-CH₂OH (worin R⁸ OR⁷ oder OH bedeutet), gegebenenfalls Abspaltung der Schutzgruppe R⁷ zu Verbindungen der Formel R¹-NH-CH₂-CH(OH)-CH₂OH, Reaktion mit einem Derivat der Kohlensäure wie Diethylcarbonat zu 3-R¹-5-hydroxymethyl-2-oxazolidinonen und Umwandlung der Hydroxymethylgruppe in eine CH₂Z-Gruppe, z.B. mit SOCl₂, SOBr₂, Methansulfonylchlorid oder p-Toluolsulfonylchlorid. Die Verbindungen der Formel Y-B (III) sind in der Regel bekannt oder in Analogie zu bekannten Verbindungen herstellbar.

Verbindungen der Formel I können ferner erhalten werden durch Reaktion einer Verbindung der Formel IV (oder eines reaktionsfähigen Derivats davon) mit einem reaktiven Derivat der Kohlensäure.

Als Kohlensäurederivate eignen sich insbesondere Dialkylcarbonate wie Diethylcarbonat, ferner auch Chlorameisensäurealkylester wie Ethylchlorformiat. Bevorzugt dient das Kohlensäurederivat, das zweckmäßig im Überschuß eingesetzt wird, auch als Lösungs- bzw. Suspensionsmittel. Es kann aber auch eines der angegebenen Lösungsmittel anwesend sein, sofern es bei dieser Umsetzung inert ist. Weiterhin empfiehlt sich der Zusatz einer Base, insbesondere eines Alkalimetallalkoholats wie Kalium-tert.-butylat. Man arbeitet zweckmäßig bei Reaktionstemperaturen zwischen 0 und 150°, vorzugsweise zwischen 70 und 120°.

Die Ausgangsstoffe der Formel IV sind in der Regel neu. Sie sind z.B. erhältlich durch Funktionalisierung der oben genannten Verbindungen der Formel R¹-NH-CH₂-CH(OH)-CH₂OH zu Verbindungen der Formel R¹-NH-CH₂-CH(OH)-CH₂-Z und Reaktion mit Verbindungen der Formel B-Y (III).

Zur Herstellung von Verbindungen der Formel I, worin R¹ eine Guanidinophenylgruppe bedeutet, kann man eine entsprechende Aminophenylverbindung mit einem amidinierenden Mittel behandeln. Als amidinierendes Mittel ist 1-Amidino-3,5-dimethylpyrazol bevorzugt, das insbesondere in Form seines Nitrats eingesetzt wird. Man arbeitet zweckmäßig unter Zusatz einer Base wie Triethylamin oder Ethyl-diisopropylamin in einem inerten Lösungsmittel oder Lösungsmittelgemisch, z.B. Wasser/Dioxan bei Temperaturen zwischen 0 und 120°, vorzugsweise 60 und 120°.

Weiterhin ist es möglich, in einer Verbindung der Formel I einen oder beide der Reste R¹ und/oder B in (einen) andere(n) Rest(e) R¹ und/oder B umzuwandeln.

Insbesondere kann man Cyangruppen zu Aminomethylgruppen reduzieren oder in Amidinogruppen umwandeln, Carboxylgruppen verestern, Estergruppen spalten, Benzylgruppen hydrogenolytisch entfernen, Aminomethylgruppen in Guanidinomethylgruppen überführen.

Eine Reduktion von Cyangruppen zu Aminomethylgruppen gelingt zweckmäßigerweise durch katalytische Hydrierung, z. B. an Raney-Nickel bei Temperaturen zwischen 0 und 100°, vorzugsweise 10 und 30°, und Drucken zwischen 1 und 200 bar, vorzugsweise bei Normaldruck, in einem inerten Lösungsmittel, z.B. einem niederen Alkohol wie Methanol oder Ethanol, zweckmäßig in Gegenwart von Ammoniak. Arbeitet man z.B. bei etwa 20° und 1 bar, so bleiben im Ausgangsmaterial vorhandene Benzylester- oder N-Benzylgruppen erhalten. Will man diese hydrogenolytisch spalten, so verwendet man zweckmäßig einen Edelmetallkatalysator, vorzugsweise Pd-Kohle, wobei man der Lösung eine Säure wie Essigsäure sowie auch Wasser zusetzen kann.

Zur Herstellung eines Amidins der Formel I (R¹ = Amidinophenyl) kann man an ein Nitril der Formel I (R¹ = Cyanphenyl) Ammoniak anlagern. Die Anlagerung erfolgt bevorzugt mehrstufig, indem man in an sich bekannter Weise a) das Nitril mit H₂S in ein Thioamid umwandelt, das mit einem Alkylierungsminel, z.B. CH₃I, in den entsprechenden S-Alkyl-imidothioester übergeführt wird, welcher seinerseits mit NH₃ zum Amidin reagiert, b) das Nitril mit einem Alkohol, z.B. Ethanol in Gegenwart von HCl in den entsprechenden Imidoester umwandelt und diesen mit Ammoniak behandelt, oder c) das Nitril mit Lithium- bis-(trimethylsilyl)amid umsetzt und das Produkt anschließend hydrolysiert.

Analog sind die entsprechenden N-Hydroxy-amidine der Formel I (R¹ = durch HO-NH-C(=NH)substituiertes Phenyl) aus den Nitrilen erhältlich, wenn man nach a) oder b), aber mit Hydroxylamin anstelle von Ammoniak arbeitet.

Zur Veresterung kann man eine Säure der Formel I (R² = H) mit einem Überschuß eines Alkohols der Formel R²-OH (R² = A oder Benzyl) behandeln, zweckmäßig in Gegenwart einer starken Säure wie Salzsäure oder Schwefelsäure bei Temperaturen zwischen 0 und 100, vorzugsweise 20 und 50°.

Umgekehrt kann ein Ester der Formel I (R² = A oder Benzyl), in die entsprechende Säure der Formel I (R² = H) umgewandelt werden, zweckmäßig durch Solvolyse nach einer der oben angegebenen Methoden, z.B. mit NaOH oder KOH in Wasser-Dioxan bei Temperaturen zwischen 0 und 40°, vorzugsweise 10 und 30°.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Es ist auch möglich, Carbonsäuren der Formel I (R² = H) durch Umsetzung mit entsprechenden Basen in ihre Metall- oder Ammoniumsalze umzuwandeln, z.B. ihre Natrium-, Kalium- oder Calciumsalze.

Die Verbindungen der Formel I enthalten ein oder mehrere chirale Zentren und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch-aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren wie β-Camphersulfonsäure.

Vorteilhaft ist auch eine Enantiomerentrennung mit Hilfe einer mit einem optisch aktiven Trennmittel (z.B. Dinitrobenzoyl-phenyl-glycin) gefüllten Säule; als Laufmittel eignet sich z.B. ein Gemisch Hexan/Isopropanol/Acetonitril.

Natürlich ist es auch möglich, optisch-aktive Verbindungen der Formel I nach den oben beschriebenen Methoden zu erhalten, indem man Ausgangsstoffe (z.B. solche der Formel II) verwendet, die bereits optisch-aktiv sind.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate.

Für die Applikation als Inhalations-Spray können Sprays verwendet werden, die den Wirkstoff entweder gelöst oder suspendiert in einem Treibgasgemisch enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Pharmaka, insbesondere aber in Analogie zu den in der EP-A-459256 beschriebenen Verbindungen verabreicht, vorzugsweise in Dosierungen zwischen etwa 5 mg und 1 g, insbesondere zwischen 50 und 500 mg pro Dosierungseinheit. Die tägliche Dosierung liegt vorzugsweise zwischen etwa 0,1 und 20mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, extrahiert mit Ethylacetat oder Dichlormethan, trennt ab, trocknet die organische Phase über Natriumsulfat, dampft ein und reinigt durch Chromatographie an Kieselgel und/oder Kristallisation.

### Beispiel 1

Zu einer Lösung von 1,7 g Na-p-methoxycarbonylmethyl-phenolat [erhältlich durch Überführung von p-Hydroxybenzylcyanid in die entsprechende Carbonsäure, Veresterung mit Methanol zu p-Methoxycarbonylmethylphenol und anschließende Umwandlung in das Phenolat] in 20 ml Dimethylformamid (DMF) gibt man 1 Äquivalent NaH und rührt 30 Min. bei Raumtemperatur. Danach fügt man 3,0 g 3-p-Cyanphenyl-5-methansulfonyloxy-methyl-oxazolidin-2-on ("A") [erhältlich durch Reaktion von p-Aminobenzonitril mit 2,3-Epoxypropan-1-ol zu p-(N-2,3-Dihydroxypropylamino)-benzonitril, Umsetzung mit Diethylcarbonat in Gegenwart von K-tert.-butylat zu 3-p-Cyanphenyl-5-hydroxymethyl-oxazolidin-2-on und anschließende Veresterung mit Methansulfonylchlorid], gelöst in 10 ml DMF, hinzu und rührt erneut 15 Min. bei Raumtemperatur. Nach Entfernung des Lösungsmittels und üblicher Aufarbeitung erhält man das 3-p-Cyanphenyl-5-(p-methoxycarbonyl-methyl-phenoxymethyl)-oxazolidin-2-on, F. 114-115°.

Analog erhält man durch Umsetzung von "A"
mit Na-o-methoxycarbonylmethyl-phenolat das 3-p-Cyan-phenyl-5-(o-methoxycarbonyl-methyl-phenoxymethyl)-oxazolidin-2-on, M⁺ + 1 = 366;
mit Na-m-methoxycarbonylmethyl-phenolat das 3-p-Cyan-phenyl-5-(m-methoxycarbonyl-methyl-phenoxymethyl)-oxazolidin-2-on, F. 129-130°;
mit Na-2,4-bis-(methoxycarbonylmethyl)-phenolat das 3-p-Cyan-phenyl-5-[2,4-bis-(methoxycarbonyl-methyl)-phenoxymethyl]-oxazolidin-2-on;
mit Na-2,5-bis-(methoxycarbonylmethyl)-phenolat das 3-p-Cyan-phenyl-5-[2,5-bis-(methoxycarbonyl-methyl)-phenoxymethyl]-oxazolidin-2-on;
mit Na-2,6-bis-(methoxycarbonylmethyl)-phenolat das 3-p-Cyan-phenyl-5-[2,6-bis-(methoxycarbonyl-methyl)-phenoxymethyl]-oxazolidin-2-on;
mit Na-3,4-bis-(methoxycarbonylmethyl)-phenolat das 3-p-Cyan-phenyl-5-[3,4-bis-(methoxycarbonyl-methyl)-phenoxymethyl]-oxazolidin-2-on;
mit Na-3,5-bis-(methoxycarbonylmethyl)-phenolat das 3-p-Cyan-phenyl-5-[3,5-bis-(methoxycarbonyl-methyl)-phenoxymethyl]-oxazolidin-2-on;

### Beispiel 2

Eine Lösung von 0,9 g 3-p-Cyanphenyl-5-(p-methoxycarbonyl-methylphenoxymethyl)-oxazolidin-2-on (F. 114-115°) in 40 ml l0%iger methanolischer NH₃-Lösung wird an 0,6 g Raney-Ni bei Raumtemperatur und 1 bar bis zum Ende der H₂-Aufnahme hydriert. Nach Filtrieren und Eindampfen erhält man durch übliche Aufarbeitung 3-p-Aminomethylphenyl-5-(p-methoxycarbonyl-methyl-phenoxymethyl)-oxazolidin-2-on.

Analog erhält man durch Hydrierung der entsprechenden Nitrile:
3-p-Aminomethyl-phenyl-5-(o-methoxycarbonyl-methyl-phenoxymethyl)-oxazolidin-2-on;
3-p-Aminomethyl-phenyl-5-(m-methoxycarbonyl-methyl-phenoxymethyl)-oxazolidin-2-on;
3-p-Aminomethyl-phenyl-5-[2,4-bis-(methoxycarbonyl-methyl)-phenoxymethyl]-oxazolidin-2-on;
3-p-Aminomethyl-phenyl-5-[2,5-bis-(methoxycarbonyl-methyl)-phenoxymethyl]-oxazolidin-2-on;
3-p-Aminomethyl-phenyl-5-[2,6-bis-(methoxycarbonyl-methyl)-phenoxymethyl]-oxazolidin-2-on;
3-p-Aminomethyl-phenyl-5-[3,4-bis-(methoxycarbonyl-methyl)-phenoxymethyl]-oxazolidin-2-on;
3-p-Aminomethyl-phenyl-5-[3,5-bis-(methoxycarbonyl-methyl)-phenoxymethyl]-oxazolidin-2-on;

### Beispiel 3

Man löst 2,4 g 3-p-Aminomethyl-phenyl-5-(p-methoxycarbonyl-methylphenoxymethyl)-oxazolidin-2-on in 20 ml Dichlormethan, gibt 12 ml Trifluoressigsäure hinzu und rührt 20 Min. bei Raumtemperatur.

Nach Eindampfen und üblicher Aufarbeitung erhält man 3-p-Aminomethylphenyl-5-(p-carboxy-methyl-phenoxymethyl)-oxazolidin-2-on.

Analog erhält man durch Verseifung der entsprechenden Ester die folgenden Carbonsäuren:
3-p-Cyanphenyl-5-(p-carboxy-methyl-phenoxymethyl)-oxazolidin-2-on;
3-p-Cyan-phenyl-5-(o-carboxy-methyl-phenoxymethyl)-oxazolidin-2-on;
3-p-Cyan-phenyl-5-(m-carboxy-methyl-phenoxymethyl)-oxazolidin-2-on;
3-p-Cyan-phenyl-5-[2,4-bis-(carboxy-methyl)-phenoxy-methyl]-oxazolidin-2-on;
3-p-Cyan-phenyl-5-[2,5-bis-(carboxy-methyl)-phenoxy-methyl]-oxazolidin-2-on;
3-p-Cyan-phenyl-5-[2,6-bis-(carboxy-methyl)-phenoxymethyl]-oxazolidin-2-on;
3-p-Cyan-phenyl-5-[3,4-bis-(carboxy-methyl)-phenoxymethyl]-oxazolidin-2-on;
3-p-Cyan-phenyl-5-[3,5-bis-(carboxy-methyl)-phenoxymethyl]-oxazolidin-2-on;
3-p-Aminomethyl-phenyl-5-(p-carboxy-methyl-phenoxymethyl)-oxazolidin-2-on;
3-p-Aminomethyl-phenyl-5-(o-carboxy-methyl-phenoxymethyl)-oxazolidin-2-on;
3-p-Aminomethyl-phenyl-5-(m-carboxy-methyl-phenoxymethyl)-oxazolidin-2-on;
3-p-Aminomethyl-phenyl-5-[2,4-bis-(carboxy-methyl)-phenoxy-methyl]-oxazolidin-2-on;
3-p-Aminomethyl-phenyl-5-[2,5-bis-(carboxy-methyl)-phenoxymethyl]-oxazolidin-2-on;
3-p-Aminomethyl-phenyl-5-[2,6-bis-(carboxy-methyl)-phenoxymethyl]-oxazolidin-2-on;
3-p-Aminomethyl-phenyl-5-[3,4-bis-(carboxy-methyl)-phenoxymethyl]-oxazolidin-2-on;
3-p-Aminomethyl-phenyl-5-[3,5-bis-(carboxy-methyl)-phenoxymethyl]-oxazolidin-2-on;

### Beispiel 4

Zu einer Lösung von 0,6 g 3-p-Aminomethyl-phenyl-5-(p-carboxy-methylphenoxymethyl)-oxazolidin-2-on in 20 ml THF fügt man 20 ml 20%ige NaOH-Lösung hinzu und rührt 24 Std. bei Raumtemperatur. Man erhält 3-p-Aminomethyl-phenyl-5-(p-carboxy-methyl-phenoxymethyl)-oxazolidin-2-on-Na-Salz, F. 286-287°.

### Beispiel 5

Eine Lösung von 0,2 g 1-Amidino-3,5-dimethylpyrazol-nitrat in 17 ml Dioxan und 5 ml Wasser wird mit 0,17 ml Ethyldiisopropylamin versetzt und 15 Min. gerührt. Anschließend gibt man 0,4 g 3-p-Aminomethylphenyl-5-(p-methoxycarbonyl-methyl-phenoxymethyl)-oxazolidin-2-on hinzu, kocht das Gemisch 30 Std., dampft ein und arbeitet wie üblich auf. Man erhält 3-p-Guanidinomethyl-phenyl-5-(p-methoxycarbonyl-methylphenoxymethyl)-oxazolidin-2-on.

Analog erhält man
mit 3-p-Aminomethyl-phenyl-5-(o-methoxycarbonyl-methyl-phenoxymethyl)-oxazolidin-2-on das 3-p-Guanidinomethyl-phenyl-5-(o-methoxycarbonyl-methyl-phenoxymethyl)-oxazolidin-2-on;
mit 3-p-Aminomethyl-phenyl-5-(m-methoxycarbonyl-methylphenoxymethyl)-oxazolidin-2-on das 3-p-Guanidinomethyl-phenyl-5-(m-methoxycarbonyl-methyl-phenoxymethyl)-oxazolidin-2-on;
mit 3-p-Aminomethyl-phenyl-5-[2,4-bis-(methoxycarbonyl-methyl)-phenoxymethyl]-oxazolidin-2-on das 3-p-Guanidinomethyl-phenyl-5-[2,4-bis-(methoxycarbonyl-methyl)-phenoxymethyl]-oxazolidin-2-on;
mit 3-p-Aminomethyl-phenyl-5-[2,5-bis-(methoxycarbonyl-methyl)-phenoxymethyl]-oxazolidin-2-on das 3-p-Guanidinomethyl-phenyl-5-[2,5-bis-(methoxycarbonyl-methyl)-phenoxymethyl]-oxazolidin-2-on;
mit 3-p-Aminomethyl-phenyl-5-[2,6-bis-(methoxycarbonyl-methyl)-phenoxymethyl]-oxazolidin-2-on das 3-p-Guanidinomethyl-phenyl-5-[2,6-bis-(methoxycarbonyl-methyl)-phenoxymethyl]-oxazolidin-2-on;
mit 3-p-Aminomethyl-phenyl-5-[3,4-bis-(methoxycarbonyl-methyl)-phenoxymethyl]-oxazolidin-2-on das 3-p-Guanidinomethyl-phenyl-5-[3,4-bis-(methoxycarbonyl-methyl)-phenoxymethyl]-oxazolidin-2-on;
mit 3-p-Aminomethyl-phenyl-5-[3,5-bis-(methoxycarbonyl-methyl)-phenoxymethyl]-oxazolidin-2-on das 3-p-Guanidinomethyl-phenyl-5-[3,5-bis-(methoxycarbonyl-methyl)-phenoxymethyl]-oxazolidin-2-on;

### Beispiel 6

Man leitet in eine Lösung von 1,2 g 3-p-Cyanphenyl-5-(p-methoxycarbonyl-methyl-phenoxy-methyl)-oxazolidin-2-on [erhältlich gemäß Beispiel 1] in 50 ml Pyridin und 7 ml Triethylamin bei -10° H₂S-Gas ein. Anschließend rührt man 14 Std. bei Raumtemperatur, dampft ein, löst den Rückstand in 50 ml Aceton und versetzt mit 9 ml Methyliodid. Nachdem man erneut 6 Std. gerührt hat, filtriert man ab, wäscht den Rückstand mit 5 ml Aceton, löst denselben in 30 ml Methanol, gibt 4,6 g Ammoniumacetat hinzu und rührt 24 Std. bei Raumtemperatur. Nach üblicher Aufarbeitung erhält man 3-p-Amidino-phenyl-5-(p-methoxycarbonyl-methyl-phenoxymethyl-)oxazolidin-2-on, (Semi-Hydroiodid), F. 151-152°.

Analog erhält man
aus 3-p-Cyan-phenyl-5-(o-methoxycarbonyl-methyl-phenoxy-methyl)-oxazolidin-2-on: 3-p-Amidino-phenyl-5-(o-methoxycarbonyl-methyl-phenoxy-methyl)-oxazolidin-2-on (Hydroiodid), M⁺ +1 = 384;
aus 3-p-Cyan-phenyl-5-(m-methoxycarbonyl-methyl-phenoxy-methyl)-oxazolid in-2-on: 3-p-Amidino-phenyl-5-(m-methoxycarbonyl-methyl-phenoxy-methyl)-oxazolidin-2-on (Hydroiodid), M⁺ + 1 = 384;
aus 3-p-Cyan-phenyl-5-[2,4-bis-(methoxycarbonyl-methyl)-phenoxymethyl]-oxazolidin-2-on: 3-p-Amidino-phenyl-5-[2,4-bis-(methoxycarbonyl-methyl)-phenoxymethyl]-oxazolidin-2-on;
aus 3-p-Cyan-phenyl-5-[2,5-bis-(methoxycarbonyl-methyl)-phenoxymethyl]-oxazolidin-2-on: 3-p-Amidino-phenyl-5-[2,5-bis-(methoxycarbonyl-methyl)-phenoxymethyl]-oxazolidin-2-on;
aus 3-p-Cyan-phenyl-5-[2,6-bis-(methoxycarbonyl-methyl)-phenoxymethyl]-oxazolidin-2-on: 3-p-Amidino-phenyl-5-[2,6-bis-(methoxycarbonyl-methyl)-phenoxymethyl]-oxazolidin-2-on;
aus 3-p-Cyan-phenyl-5-[3,4-bis-(methoxycarbonyl-methyl)-phenoxymethyl]-oxazolidin-2-on: 3-p-Amidino-phenyl-5-[3,4-bis-(methoxycarbonyl-methyl)-phenoxymethyl]-oxazolidin-2-on;
aus 3-p-Cyan-phenyl-5-[3,5-bis-(methoxycarbonyl-methyl)-phenoxymethyl]-oxazolidin-2-on: 3-p-Amidino-phenyl-5-[3,5-bis-(methoxycarbonyl-methyl)-phenoxymethyl]-oxazolidin-2-on;

### Beispiel 7

Analog Beispiel 3 erhält man durch Verseifung der entsprechenden Ester aus Beispiel 6 die folgenden Carbonsäuren:
3-p-Amidino-phenyl-5-(p-carboxy-methyl-phenoxy-methyl)-oxazolidin-2-on, F. 281°.
3-p-Amidino-phenyl-5-(o-carboxy-methyl-phenoxy-methyl)-oxazolidin-2-on, F. 274°;
3-p-Amidino-phenyl-5-(m-carboxy-methyl-phenoxy-methyl)-oxazolidin-2-on (Hydrochlorid), F. 271°;
3-p-Amidino-phenyl-5-[2,4-bis-(carboxy-methyl)-phenoxy-methyl]-oxazolidin-2-on;
3-p-Amidino-phenyl-5-[2,5-bis-(carboxy-methyl)-phenoxy-methyl]-oxazolidin-2-on;
3-p-Amidino-phenyl-5-[2,6-bis-(carboxy-methyl)-phenoxy-methyl]-oxazolidin-2-on;
3-p-Amidino-phenyl-5-[3,4-bis-(carboxy-methyl)-phenoxy-methyl]-oxazolidin-2-on;
3-p-Amidino-phenyl-5-[3,5-bis-(carboxy-methyl)-phenoxy-methyl]-oxazolidin-2-on;

### Beispiel 8

Analog Beispiel 3 erhält man durch Verseifung der entsprechenden Ester aus Beispiel 5 die folgenden Carbonsäuren:
3-p-Guanidinomethyl-phenyl-5-(p-carboxy-methyl-phenoxy-methyl)-oxazolidin-2-on, F. > 300°;
3-p-Guanidinomethyl-phenyl-5-(o-carboxy-methyl-phenoxy-methyl)-oxazolidin-2-on;
3-p-Guanidinomethyl-phenyl-5-(m-carboxy-methyl-phenoxy-methyl)-oxazolidin-2-on;
3-p-Guanidinomethyl-phenyl-5-[2,4-bis-(carboxy-methyl)-phenoxy-methyl]-oxazolidin-2-on;
3-p-Guanidinomethyl-phenyl-5-[2,5-bis-(carboxy-methyl)-phenoxy-methyl]-oxazolidin-2-on;
3-p-Guanidinomethyl-phenyl-5-[2,6-bis-(carboxy-methyl)-phenoxy-methyl]-oxazolidin-2-on;
3-p-Guanidinomethyl-phenyl-5-[3,4-bis-(carboxy-methyl)-phenoxy-methyl]-oxazolidin-2-on;
3-p-Guanidinomethyl-phenyl-5-[3,5-bis-(carboxy-methyl)-phenoxy-methyl]-oxazolidin-2-on;

Die folgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, lyophilisiert und steril verschlossen. Jedes Injektionsglas enthält 5 mg Wirkstoff.

### Beispiel B: Suppositorien

Man schmilzt ein Gemisch von 20 mg eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ x 2 H₂O, 28,48 g Na₂HPO₄ × 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedinungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird in Ampullen abgefüllt, unter aseptischen Bedingungen lyophilisiert und steril verschlossen. Jede Ampulle enthält 10 mg Wirkstoff.

## Patentansprüche

1. Oxazolidinonderivate der Formel I 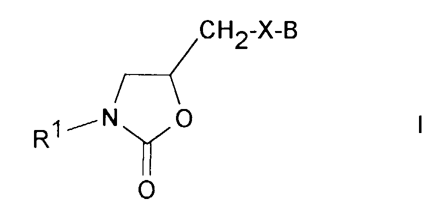 worin
R¹ einen einfach durch H₂N-CH₂-, (A)₂N-CH₂-, H₂N-C(=NH)-, H₂N-C(=NH)-NH-, H₂N-C(=NH)-NH-CH₂-, HO-NH-C(=NH)- oder HO-NH-C(=NH)-NH substituierten Phenylrest,
X O,
B 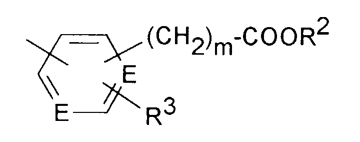
A Alkyl mit 1-6 C-Atomen,
R² H, A, Li, Na, K, NH₄ oder Benzyl,
R³ H oder (CH₂)ₙ-COOR²,
E CH,
m 1, 2 oder 3 und
n 0, 1, 2 oder 3 bedeuten,
sowie deren physiologisch unbedenkliche Salze.

2. Verbindungen nach Anspruch 1
(a) 3-p-Amidino-phenyl-5-(p-carboxymethyl-phenoxy-methyl)-oxazolidin-2-on;
(b) 3-p-Amidino-phenyl-5-(p-methoxycarbonylmethyl-phenoxy-methyl)-oxazolidin-2-on;
(c) 3-p-Aminomethyl-phenyl-5-(p-carboxymethyl-phenoxy-methyl)-oxazolidin-2-on-Natriumsalz;
(d) 3-p-Guanidinomethyl-phenyl-5-(p-carboxymethyl-phenoxy-methyl)-oxazolidin-2- on.

3. Eine enantiomere Verbindung der Formel I gemäß Anspruch 1 oder eines seiner Salze.

4. Verfahren zur Herstellung von Verbindungen der Formel I gemäß Anspruch 1 sowie deren Salzen, dadurch gekennzeichnet, daß man eine Verbindung der Formel II 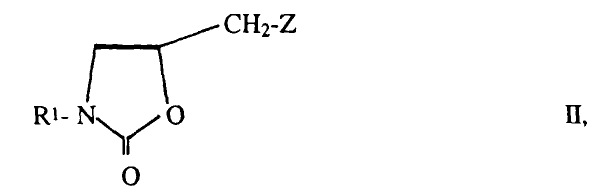 worin
R¹ die in Anspruch 1 angegebene Bedeutung hat
und
Z Cl, Br, I, OH oder eine reaktionsfähig veresterte OH-Gruppe bedeutet,
mit einer Verbindung der Formel III
Y-B III,
worin
B die in Anspruch 1 angegebene Bedeutung hat
und
Y OH, SH, NH₂, NAH oder einen aus OH oder SH ableitbaren salzartigen Rest bedeuten,
umsetzt, oder daß man
eine Verbindung der Formel IV
R¹-NH-CH₂-CH(OH)-CH₂-X-B IV,
worin
R¹, B und X die in Anspruch 1 angegebenen Bedeutungen haben, oder eines ihrer reaktionsfähigen Derivate mit einem reaktiven Derivat der Kohlensäure umsetzt,
oder daß man zur Herstellung einer Guanidinoverbindung der Formel I (R¹ = ein einfach durch H₂N-C(=NH)-NH- substituierter Phenylrest) eine Aminoverbindung entsprechend der Formel I, die jedoch anstelle des Restes R¹ eine Aminophenylgruppe enthält, mit einem amidinierenden Mittel behandelt,
oder daß man eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder beide Reste R¹ und/oder B in (einen) andere(n) Rest(e) R¹ und/oder B umwandelt und/oder eine Verbindung der Formel I durch Behandeln mit einer Säure oder einer Base in eines ihrer Salze überführt.

5. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

6. Pharmazeutische Zubereitung, gekennzeichnet durch einen Gehalt an mindestens einer Verbindung der Formel I nach Anspruch 1 und/oder einem ihrer physiologisch unbedenklichen Salze.

7. Verwendung von Verbindungen der Formel I nach Anspruch 1 oder von deren physiologisch unbedenklichen Salzen zur Herstellung eines Arzneimittels.

8. Verwendung von Verbindungen der Formel I nach Anspruch 1 oder von deren physiologisch unbedenklichen Salzen bei der Bekämpfung von Thrombosen, Herzinfarkten, Apoplexie, Osteoporose, Arteriosklerose, Entzündungen und/oder Tumoren.

## Claims

1. Oxazolidinone derivatives of the formula I 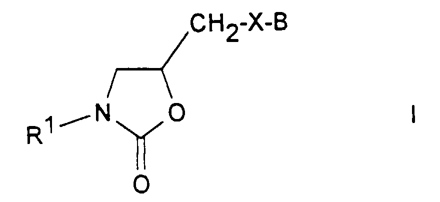 in which
R¹ is a phenyl radical which is monosubstituted by H₂N-CH₂-, (A)₂N-CH₂-, H₂N-C(=NH)-, H₂N-C(=NH)-NH-, H₂N-C(=NH)-NH-CH₂-, HO-NH-C(=NH)- or HO-NH-C(=NH)-NH-,
X is O,
B is 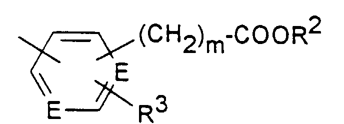
A is alkyl having from 1-6 C atoms,
R² is H, A, Li, Na, K, NH₄ or benzyl,
R³ is H or (CH₂)ₙ-COOR²,
E is CH,
m is 1, 2 or 3, and
n is 0, 1, 2 or 3,
and physiologically harmless salts thereof.

2. Compounds according to Claim 1
(a) 3-p-Amidinophenyl-5-(p-carboxymethylphenoxymethyl)oxazolidin-2-one;
(b) 3-p-Amidinophenyl-5-(p-methoxycarbonylmethylphenoxymethyl)oxazolidin-2-one;
(c) 3-p-Aminomethylphenyl-5-(p-carboxymethylphenoxymethyl)oxazolidin-2-one sodium salt;
(d) 3-p-Guanidinomethylphenyl-5-(p-carboxymethylphenoxymethyl)oxazolidin-2-one.

3. An enantiomeric compound of the formula I according to Claim 1 or one of its salts.

4. Process for preparing compounds of the formula I according to Claim 1, and also their salts, characterized in that a compound of the formula II 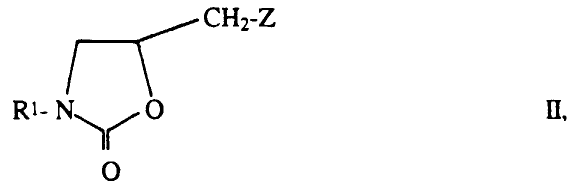 in which
R¹ has the meaning given in Claim 1,
and
Z is Cl, Br, I, OH or a reactive esterified OH group,
is reacted with a compound of the formula III
Y-B III,
in which
B has the meanings given in Claim 1,
and
Y is OH, SH, NH₂, NAH or a salt-like radical which can be derived from OH or SH,
or in that
a compound of the formula IV
R¹-NH-CH₂-CH(OH)-CH₂-X-B IV,
in which
R¹, B and X have the meanings given in Claim 1, or one of its reactive derivatives, is reacted with a reactive derivative of carbonic acid,
or in that, in order to prepare a guanidino compound of the formula I (R¹ = a phenyl radical which is monosubstituted by H₂N-C(=NH)-NH-), an amino compound corresponding to the formula I, which compound, however, contains an aminophenyl group in place of the radical R¹, is treated with an amidinating agent,
or in that a compound of the formula I is liberated from one of its functional derivatives by treatment with a solvolysing or hydrogenolysing agent,
and/or in that, in a compound of the formula I, one or both of the radicals R¹ and/or B is/are converted into (an) other radical(s) R¹ and/or B, and/or a compound of the formula I is converted into one of its salts by treatment with an acid or a base.

5. Process for producing pharmaceutical preparations, characterized in that a compound of the formula I according to Claim 1 and/or one of its physiologically harmless salts is brought into a suitable dosage form together with at least one solid, liquid or semiliquid excipient or auxiliary substance.

6. Pharmaceutical preparation, characterized by a content of at least one compound of the formula I according to Claim 1 and/or one of its physiologically harmless salts.

7. Use of compounds of the formula I according to Claim 1, or of their physiologically harmless salts, for producing a medicament.

8. Use of compounds of the formula I according to Claim 1, or of their physiologically harmless salts, in the control of thromboses, cardiac infarctions, stroke, osteoporosis, arteriosclerosis, inflammations and/or tumours.

## Revendications

1. Dérivés de l'oxazolidinone de formule I 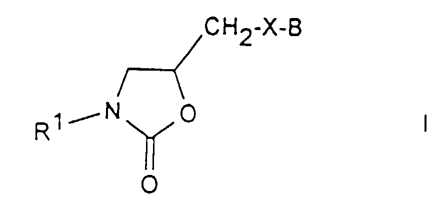 où
R¹ représente un reste phényle monosubstitué par
H₂N-CH₂-, (A) ₂N-CH₂-, H₂N-C(=NH)-, H₂N-C(=NH)-NH-,
H₂N-C(=NH)-NH-CH₂-, HO-NH-C(=NH)- ou HO-NH-C(=NH)-NH-,
X O,
B 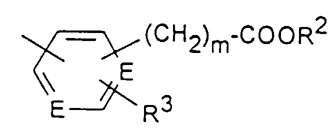
A un alkyle comportant 1 à 6 atomes de C,
R² H, A, Li, Na, K, NH₄ ou le benzyle,
R³ H ou (CH₂)ₙ-COOR²,
E CH,
m est égal à 1, 2 ou 3 et
n est égal à 0, 1, 2 ou 3,
ainsi que leurs sels physiologiquement acceptables.

2. Les composés selon la revendication 1
(a) la 3-p-amidinophényl-5-(p-carboxyméthylphénoxyméthyl)oxazolidin-2-one,
(b) la 3-p-amidinophényl-5-(p-méthoxycarbonylméthylphénoxyméthyl)oxazolidin-2-one,
(c) le sel de sodium de la 3-p-aminométhylphényl-5-(p-carboxyméthylphénoxyméthyl)oxazolidin-2-one,
(d) la 3-p-guanidinométhylphényl-5-(p-carboxyméthylphénoxyméthyl)oxazolidin-2-one.

3. Un composé énantiomère de formule I selon la revendication 1 ou l'un de ses sels.

4. Procédé pour la préparation des composés de formule I selon la revendication 1, ainsi que de leurs sels, caractérisé en ce que l'on fait réagir un composé de formule Il 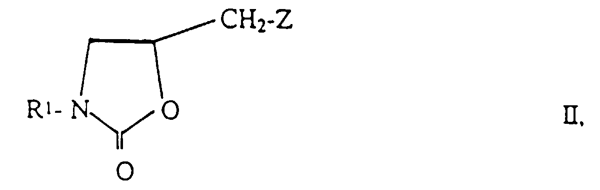 où
R¹ a la signification indiquée dans la revendication 1
et
Z représente C1, Br, I, OH ou un groupe OH estérifié réactif,
avec un composé de formule III
Y-B III
où
B a la signification indiquée dans la revendication 1
et
Y représente OH, SH, NH₂, NAH ou un reste du genre sel dérivable de OH ou de SH
ou
en ce que l'on fait réagir un composé de formule IV
R¹-NH-CH₂-CH(OH)-CH₂-X-B IV
où
R¹, B et X ont les significations indiquées dans la revendication 1, ou l'un de ses dérivés réactifs, avec un dérivé réactif de l'acide carbonique,
ou en ce que l'on traite, pour la préparation d'un composé guanidino de formule I (R¹ = un reste phényle monosubstitué par H₂N-C(=NH)-NH-), un composé aminé correspondant à la formule I, mais contenant à la place du reste R¹ un groupe aminophényle, avec un agent amidinant,
ou en ce que l'on libère un composé de formule I de l'un de ses dérivés fonctionnels par traitement avec un agent solvolysant ou hydrogénolysant,
et/ou en ce que l'on transforme dans un composé de formule I le ou les deux restes R¹ et/ou B en un ou deux autres restes R¹ et/ou B et/ou en ce que l'on transforme un composé de formule I en l'un de ses sels par traitement avec un acide ou une base.

5. Procédé pour la fabrication de préparations pharmaceutiques, caractérisé en ce que l'on met sous la forme d'un dosage approprié un composé de formule I selon la revendication 1 et/ou l'un de ses sels physiologiquement acceptables avec au moins un support ou un adjuvant solide, liquide ou semi-liquide.

6. Préparation pharmaceutique caractérisée en ce qu'elle contient au moins un composé de formule I selon la revendication 1 et/ou l'un de ses sels physiologiquement acceptables.

7. Utilisation des composés de formule I selon la revendication 1 ou de leurs sels physiologiquement acceptables pour la fabrication d'un médicament.

8. Utilisation des composés de formule I selon la revendication 1 ou de leurs sels physiologiquement acceptables pour lutter contre les thromboses, les infarctus du myocarde, l'apoplexie, l'ostéoporose, l'artériosclérose, les inflammations et/ou les tumeurs.
